Europäisches Patentamt

(19) European Patent Office

Office européen des brevets

(11) Publication number : **0 467 660 A2**

(12) **EUROPEAN PATENT APPLICATION**

(21) Application number : **91306490.3**

(22) Date of filing : **17.07.91**

(51) Int. Cl.⁵ : **A61K 31/035, A61K 7/06**

(30) Priority : **20.07.90 JP 190563/90**
**09.07.91 JP 168268/91**

(43) Date of publication of application :
**22.01.92 Bulletin 92/04**

(84) Designated Contracting States :
**DE FR GB**

(71) Applicant : **Miyauchi, Yutaka**
**5-9, Shigota-machi**
**Takasaki-shi, Gunma-ken (JP)**

(72) Inventor : **Miyauchi, Yutaka**
**5-9, Shigota-machi**
**Takasaki-shi, Gunma-ken (JP)**

(74) Representative : **Perry, Robert Edward et al**
**GILL JENNINGS & EVERY 53-64 Chancery**
**Lane**
**London WC2A 1HN (GB)**

(54) Use of d-limonenes as testosterone-5-alpha-reductase inhibitor and as hair grower.

㊼   A 5α-reductase inhibitor according to the invention comprises d-limonene, which has an excellent activity inhibitory effect and is highly safe without any drawback as noted by side effects and thus is a suitable component for a hair grower.

EP 0 467 660 A2

## Background of the Invention

### 1. Field of Industrial Application

The present invention relates to a testosterone $5\alpha$-reductase (hereinafter designated simply as "$5\alpha$-reductase") inhibitor and a hair grower comprising the same.

### 2. Description of the Prior Art

Recently, increased numbers of studies have been made on a $5\alpha$-reductase inhibitor as a hair grower, and there have been many patent applications for hair growers featured by the effect of preventing loss of hair by inhibition of $5\alpha$-reductase (for example, Japanese Patent Laid-open Nos. 53,417/1984 and 53,419/1984).

According to a certain view, it is considered that physiological symptoms of, for example, male pattern baldness, psilosis, or seborrhea are caused by an increased hormonal stimulus of androgen, which results from excessive accumulation of the androgenic hormone ($5\alpha$-dihydro- testosterone) in a metabolic system. It is considered that this androgenic hormone is produced from testosterone by a reducing action of $5\alpha$-reductase, and, in particular, the androgenic hormone is produced and accumulated in some organs such as hair roots and sebaceous glands due to the action of $5\alpha$-reductase. As a result, hair-matrix cell division may be suppressed, leading to involution of the hair roots, and thereby symptoms of alopecia or baldness may be induced. It is therefore possible to prevent epilation or to promote hair-growth by inhibitory actions on the $5\alpha$-reductase. This is the reason why the $5\alpha$-reductase inhibitor is used for hair growers.

From this point of view, some compounds used for this purpose have been found. However, though they have a high $5\alpha$-reductase activity inhibitory ratio, they have drawbacks of producing side effects or causing high irritation to the skin.

## Summary of the Invention

Accordingly, an object of the present invention is to provide a $5\alpha$-reductase inhibitor which has no drawback as noted by the side effects and is highly safe.

Another object of the present invention is to provide a hair grower having an excellent hair-growth effect upon application to the human scalp.

Other objects, features and advantages of the invention will hereinafter become more readily apparent from the following description.

## Brief Description of the Drawings

Fig. 1 is a graph for explaining an enzymatic activity inhibitory ratio of sodium $\beta$-glycyrrhetinic acid.
Fig. 2 is a graph for explaining an enzymatic activity inhibitory ratio of Duke extract.
Fig. 3 is a graph for explaining an enzymatic activity inhibitory ratio of the hair grower of the present invention.
Fig. 4 is a graph for explaining an enzymatic activity inhibitory ratio of d-limonene.
Fig. 5 is a graph for explaining an enzymatic activity inhibitory ratio of swertiamarin.

## Description of the preferred Embodiment

### Example 1

Various effects of the hair grower of the present invention were studied, and the results are explained in the followings.

The present inventor has already disclosed a novel toilet lotion containing components from citrus fruits and other components (Japanese Patent Publication No. 20,123/1989). This toilet lotion is, of course, provided with the ordinary functions of a toilet lotion, e.g., astringent and cleansing effects, dampness, and lubrication of the skin. It has been confirmed that the toilet lotion gives, besides the above ordinary functions, anti-dandruff and antiprustic effects and an effect of preventing the loss of hair when rubbed into the pores of the scalp after shampooing. It has been also confirmed that some people have enjoyed the benefits of the toilet lotion, which not merely restrained the loss of their hair but also positively promoted the growth of their hair.

The toilet lotion consists of each extract from the skin of a navel orange [*Citrus sinensis L. Osbeck (Rutaceae)*], the skin of an *iyokan* [*Citrus iyo Hort. et Tanaka (Rutaceae)*], the skin of a *hassaku [Citrus hassaku* *Hort. et Tanaka (Rutaceae)]*, the skin of a sweet summer orange [*Citrus Natsudaidai var. (Rutaceae)*], the skin of a lemon, the skin of a mandarin orange, and aloe, all of said extracts being prepared by extracting with *sake*. The refined oil from the citrus fruits contains d-limonene in the amount of not less than 90% by weight. It is therefore clear that the d-limonene functions as the 5$\alpha$-reductase inhibitor and thereby contributes to prevention of the loss of hair and to promotion of the growth of hair.

The following table shows a formulation of the hair grower (toilet lotion) in a preferred form of the present invention.

## Formulation

| Components | % by weight |
|---|---|
| Extract from navel orange [*Citrus sinensis L. Osbeck (Rutaceae)*] skin | 19.75 |
| Extract from *iyokan* [*Citrus iyo Hort. et Tanaka (Rutaceae)*] skin | 14.82 |
| Extract from *hassaku* [*Citrus hassaku Hort. et Tanaka (Rutaceae)*] skin | 19.75 |
| Extract from sweet summer orange [*Citrus Natsudaidai var. (Rutaceae)*] skin | 14.82 |
| Extract from lemon skin | 14.82 |
| Extract from mandarin orange skin | 14.81 |
| Extract from aloe | 1.23 |
| pH [*1]: 4.0-6.0 | |
| Alcohol number [*2]: 0.9-1.0 | |

[*1]: determined according to Japanese Standards of Cosmetic Ingredients - General test method - Test for pH.

[*2]: determined according to The Pharmacopoeia of Japan - General test method - Test for alcohol number.

The above extracts are all those extracted with sake (first class refined *sake*) from the skin of each of the above citrus fruits or other materials. A slight variation in the proportion of each of the above components is expected in the stages of manufacturing. It was, however, confirmed that some ±5% of the proportion out of the defined range can be tolerated to ensure the same performance and safety of the composition.

## Application to the Scalp

As the symptoms identified by the loss of hair in the pilose regions of the body, especially the head, many sorts are known, such as androgenic alopecia, alopecia areata, and alopecia seborrheica. In this example, however, the hair grower containing as a major component d-limonene, which is the 5$\alpha$-reductase inhibitor according to the present invention, was applied to the scalp of dozens of persons irrespective of the sort of alopecia. The results are shown in the following tables.

| | Anti-dandruff effect *1 | | |
|---|---|---|---|
| | Excellently effective | Effective | Not effective |
| Man (58 persons) | 36 persons (62.1%) | 22 persons (37.9%) | 0 person (0%) |
| Woman (22 persons) | 10 persons (45.5%) | 12 persons (54.5%) | 0 person (0%) |
| Total (80 persons) | 46 persons (57.5%) | 34 persons (42.5%) | 0 person (0%) |

*1: Excellently : Generation of dandruff was not found
    effective    over five days after applying the hair
                 grower

    Effective:   Generation of dandruff was not found over
                 three days but found within five days
                 after applying the hair grower

    Not effective: Generation of dandruff was found within
                   three days after applying the hair
                   grower

| | Depilation preventive effect *2 | |
|---|---|---|
| | Effective | Not effective |
| Man (58 persons) | 53 persons (91.4%) | 5 persons (8.6%) |
| Woman (22 persons) | 20 persons (90.9%) | 2 persons (9.1%) |
| Total (80 persons) | 73 persons (91.3%) | 7 persons (8.7%) |

*2: Effective:   A degree of advance in loss of hair was
                 decreased after applying the hair grower

    Not effective: Depilation preventive effect was not
                   found after applying the hair grower

|  | Hair-growth effect *3 | | |
|---|---|---|---|
|  | Excellently effective | Effective | Not effective |
| Man (58 persons) | 5 persons (8.6%) | 32 persons (55.2%) | 21 persons (36.2%) |
| Woman (22 persons) | 10 persons (45.5%) | 5 persons (22.7%) | 7 persons (31.8%) |
| Total (80 persons) | 15 persons (18.8%) | 37 persons (46.3%) | 28 persons (35.0%) |

*3: Excellently effective : New hair grew in all hairless portions after applying the hair grower

Effective : New hair grew in the area larger than two third of hairless portions after applying the hair grower

Not effective: No improvement was found in a hair-growth effect after applying the hair grower

As evidenced from the above results, the hair grower of the present invention is excellently effective or effective on the anti-dandruff, prevention of depilation, and hair-growth.

Such excellent effect of the hair grower of the present invention may be due to some action of amino acid in the hair grower of the present invention (contained in the amount of 0.2% by weight in the citrus skin and the refined sake). The following is an explanation for the kinds of amino acid contained in 100 ml of the hair grower of the present invention.

| | | | |
|---|---|---|---|
| Aspartic acid | 9.84 mg | Isoleucine | 5.81 mg |
| Threonine | 7.61 mg | Leucine | 11.58 mg |
| Serine | 11.76 mg | Thyrosin | 8.46 mg |
| Glutamic acid | 8.14 mg | Phenylalanine | 7.53 mg |
| Glycine | 10.97 mg | Lysine | 6.93 mg |
| Alanine | 25.50 mg | Histidine | 3.00 mg |
| Cystine | 1.52 mg | Arginine | 21.96 mg |
| Valine | 8.55 mg | Proline | 52.22 mg |
| Methionine | 2.07 mg | Tryptophan | 1.12 mg |

EP 0 467 660 A2

## Example 2

A more detailed experiment was performed on the inhibitory actions of d-limonene on the 5α-reductase activity. As previously mentioned, it is known that testosterone is converted into 5α-dihydrotestosterone (5αDHT) having an androgenic action and thereby expresses physiological activities. 5α-Reductase is present in living bodies and functions as an enzyme which converts testosterone into 5αDHT. Therefore substances capable of impeding the activities of the enzyme will probably be prophylactics for androgenic alopecia.

From the above point of view, a system capable of measuring a testosterone 5α-reductase activity was formed, and, using the system, studies were performed on the 5α-reductase inhibitory activities of the hair grower of the present invention and of the limonene that is a constituent of the hair grower.

Two hair growers of the present invention, one aged and another fresh, and d-limonene were subjected to a test. For control groups, Duke extract and sodium β-glycyrrhetinic acid were selected as positive control groups and swertiamarin as a negative control group.

The test was performed according to the following methods.

### Preparation of a crude enzyme solution

A Wristar male rat weighing 250-300 g was sacrificed by bleeding, and its prostata was excised. The prostata was cut into fragments under ice-cooling. A 0.1 M HEPES buffer solution (pH: 7.4) containing 0.25 M sucrose (the buffer solution is hereinafter referred to as buffer solution A) was added to the fragments in the amount three times the wet weight of the fragments. Using a Potter-type homogenizer made of Teflon, the mixture was homogenized. The homogenate was filtered using a stainless mesh of a 200 μ size, and then the filtrate was centrifuged at 3000 rpm (900 xg) for ten minutes. The precipitate was suspended in 2.5 parts of buffer solution A, followed by centrifuging at 3000 rpm for five minutes. The residue recovered was suspended in buffer solution A weighing 0.8 times the wet weight of the fragments to produce a crude enzyme solution. The crude enzyme solution was stored at -70°C.

### Enzymatic reaction

The test sample (less than 10 μl) previously prepared was added to [4-$^{14}$C]-testosterone (1.5 nmol, 2.85 kBq), NADPH (0.5 μmol), and the enzyme solution (30 μl) cited above. To this was added the buffer solution A to the extent that the total volume became 100 μl, to prepare a reaction liquid. An enzymatic reaction was performed using the reaction liquid at 37°C for two hours. The enzymatic reaction was initiated by removing the reaction liquid from an ice-water-cooled bath to a 37°C warm bath. The enzymatic reaction was terminated by cooling the reaction liquid in ice-water. After completion of the reaction, 400 μl of n-hexane was added to the liquid, followed by vigorously stirring the mixture to uniformly mix. The uniformly blended mixture was centrifuged at 3000 rpm and a temperature of 4°C for five minutes to obtain a supernatant liquid. 200 μl of the supernatant liquid partially collected was evaporated under reduced pressure to a solid, to which was added 30 μl of n-hexane to redissolve. 10 μl of the liquid mixture collected in part was applied to a thin layer plate of silica gel. After drying, the deposited substance was developed using cyclohexane-ethyl acetate (1:1, v/v).

### Quantitative determination of reaction product and Enzymatic activity inhibitory ratio measuring method

A radiation activity of the developed material on the TLC plate cited above was measured by means of an AMBIS image analyzer.

Two spots shown in a chromatogram were subjected to readings of the radiation activities identified by their positions corresponding to testosterone (T) and dihydrotestosterone (DHT) respectively. The rate of conversion of testosterone into dihydrotestosterone was given by:

$$DHT/(T + DHT) \times 100\%$$

Defining the conversion rate in the presence of the negative control as 100, a percentage of the conversion rate in the presence of the sample was calculated to determine the degree of the enzymatic activity inhibitory ratio of the sample.

### Results

First, various organic solvents were investigated to seek an eluent that could clearly isolate testosterone from dihydrotestosterone on the TLC plate, and as a result it was confirmed that both materials could be clearly separated independently by the use of the aforementioned developing solvent mixture, cyclohexane-ethyl ace-

6

tate (1:1, v/v). Specifically, on the TLC plate there were the two spots representing respective radiation activities. One spot with a lower Rf value was for testosterone and another spot with a higher Rf value for dihydrotestosterone. The two spots could be thus distinguished.

Second, conditions of the enzymatic reaction were studied, and as a result it was confirmed that the reaction at 37°C for two hours was relatively effective for the conversion of testosterone into dihydrotestosterone. In this case, because the liposoluble sample was barely soluble in water, it was mixed with water by shaking at 120 rpm.

Further studies were carried out on the kinds of extracting solvents and thin layer plates, and the like.

The reaction conditions thus established are as follows:

| | |
|---|---|
| [4-$^{14}$C]-testosterone | 1.9 μl |
| NADPH | 10 μl |
| Enzyme solution | 30 μl |
| Sample | 3-10 μl |

The process was carried out by heating at 37°C for two hours a reaction liquid made up to 100 ml by the addition of a buffer solution, extracting with hexane, and developing by means of TLC.

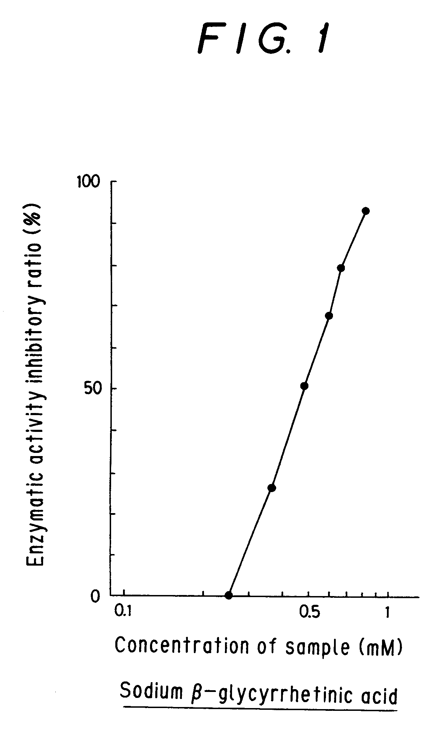

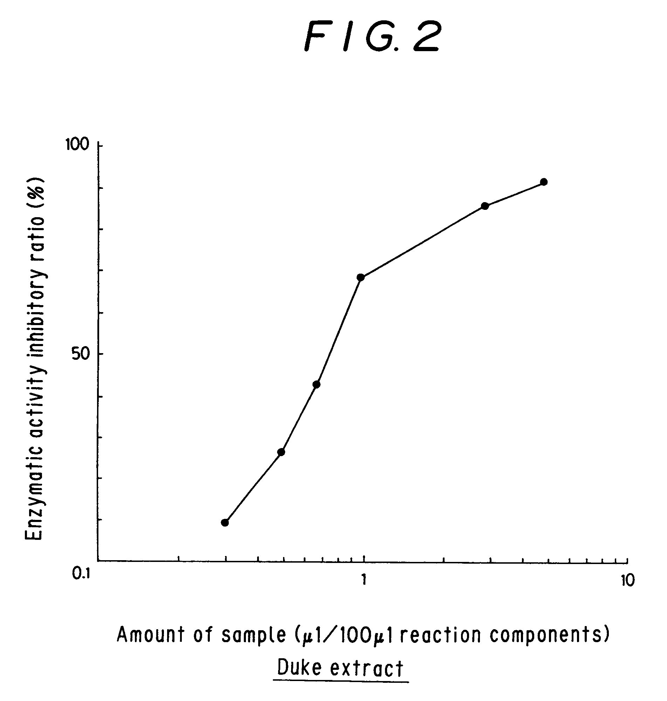

Next, studies were carried out on the inhibitory activities of sodium β-glycyrrhetinic acid and Duke extract, which were used as the positive controls. As shown in Fig. 1 and Fig. 2, the amounts of sodium β-glycyrrhetinic acid and Duke extract exhibiting a inhibitory ratio of 50% are 0.5 mM and 0.75 μl respectively. Their remarkably high inhibitory activities are thus confirmed.

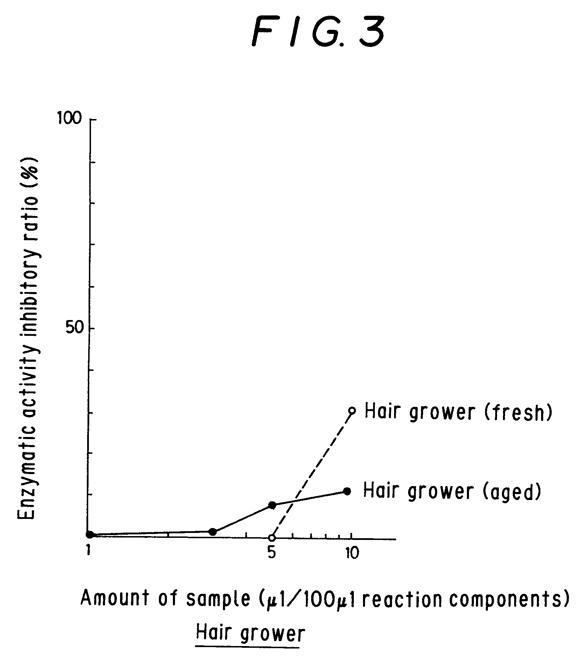

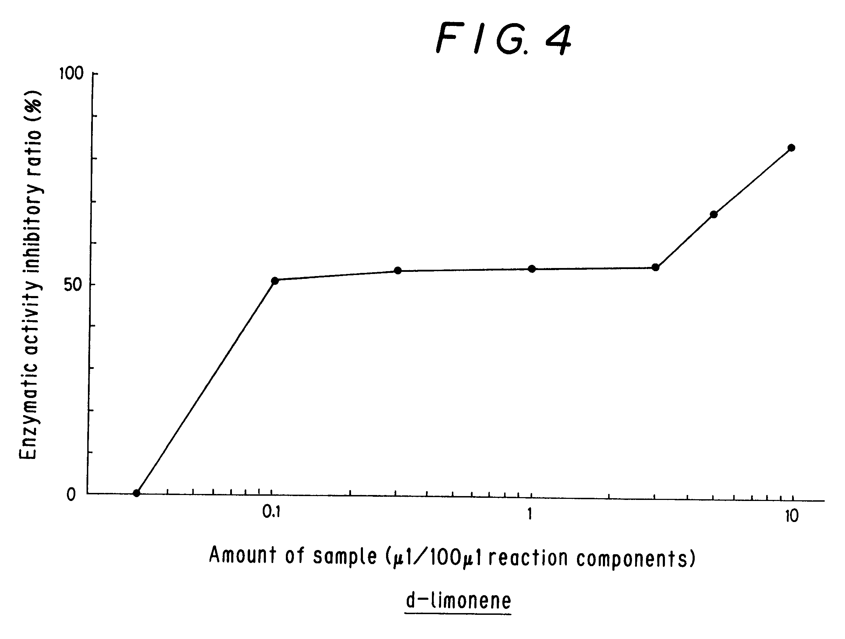

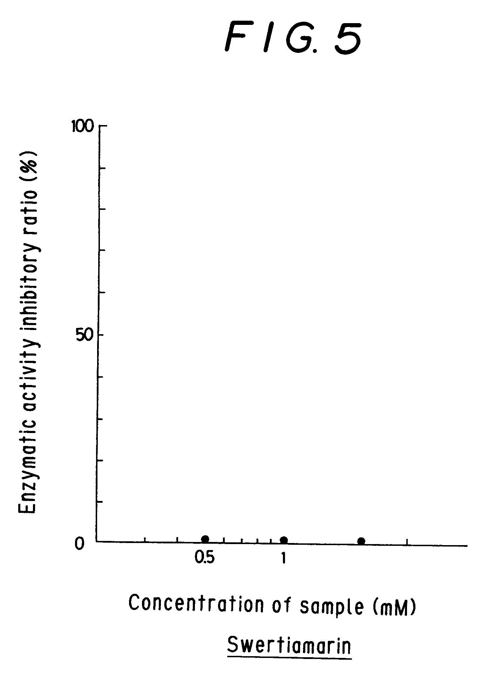

The inhibitory actions on the testosterone 5α-reductase activity of the hair growers of the present invention and of d-limonene, which is a constituent of the hair grower, were studied. As shown in Figs. 3 and 4, either of the test samples can give a considerable high inhibitory effect. Among the hair growers the aged sample gives a higher inhibitory effect than the fresh sample. On the other hand, the negative control swertiamarin has no inhibitory effect as shown in Fig. 5.

As explained above, the hair grower containing as a major component the 5α-reductase inhibitor according to the present invention provides an excellent hair-growth effect upon application to the human scalp. The hair grower is very safe in contact with the skin. Also, it can be readily produced from the skin of citrus fruits and hence has the advantages of easy preparation and reduced cost.

## Claims

1. Use of d-limonene as a testosterone 5α-reductase inhibitor.

2. Use of d-limonene as a hair grower.

3. Use of d-limonene for the manufacture of a medicament for use as a hair grower.

4. Use according to any preceding claim wherein the d-limonene is as collected from a refined oil from the skin of a citrus fruit.

5. Use according to claim 4, wherein the citrus fruit is selected from a navel orange [citrus sinensis L. Osbeck (Rutaceae)], an iyokan [citrus iyo Hort. et Tanaka (Rutaceae)], a hassaku [citrus hassaku Hort. et Tanaka (Rutaceae)], a sweet summer orange [citrus Natsudaidai var. (Rutaceae)], a lemon and a mandarin orange.

# F I G. 1

Sodium β−glycyrrhetinic acid

# F I G. 2

Amount of sample (μl/100μl reaction components)
Duke extract

FIG. 3

Hair grower

FIG. 4

d-limonene

EP 0 467 660 A2

# F I G. 5

Concentration of sample (mM)

Swertiamarin